(19) Europäisches Patentamt
European Patent Office
Office européen des brevets

(11) **EP 3 810 289 B1**

(12) **EUROPÄISCHE PATENTSCHRIFT**

(45) Veröffentlichungstag und Bekanntmachung des Hinweises auf die Patenterteilung:
**06.03.2024   Patentblatt 2024/10**

(21) Anmeldenummer: **19722125.2**

(22) Anmeldetag: **06.05.2019**

(51) Internationale Patentklassifikation (IPC):
*A61Q 19/00* (2006.01)    *A61Q 17/04* (2006.01)
*A61K 8/31* (2006.01)    *A61K 8/37* (2006.01)
*A61K 8/39* (2006.01)    *A61K 8/73* (2006.01)
*A61K 8/92* (2006.01)

(52) Gemeinsame Patentklassifikation (CPC):
**A61K 8/39; A61K 8/31; A61K 8/37; A61K 8/732; A61K 8/922; A61Q 17/04; A61Q 19/00;**
A61K 2800/31; A61K 2800/546

(86) Internationale Anmeldenummer:
**PCT/EP2019/061574**

(87) Internationale Veröffentlichungsnummer:
**WO 2019/242926 (26.12.2019 Gazette 2019/52)**

(54) **WASSERFREIE TOPISCH APPLIZIERBARE ZUBEREITUNG**

WATER-FREE TOPICALLY APPLICABLE PREPARATION

PRÉPARATION ANHYDRE APPLICABLE LOCALEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.06.2018   DE 102018209853**

(43) Veröffentlichungstag der Anmeldung:
**28.04.2021   Patentblatt 2021/17**

(73) Patentinhaber: **Beiersdorf AG**
**22529 Hamburg (DE)**

(72) Erfinder:
• **KÖHLER, Manuela**
**22527 Hamburg (DE)**
• **ZHONG, Sabilla**
**22527 Hamburg (DE)**
• **JAPP, Christina**
**21149 Hamburg (DE)**
• **GUTZKE, Doreen**
**29646 Bispingen (DE)**
• **GÖDDERTZ, Dominik**
**22549 Hamburg (DE)**

• **WISCHHÖFER, Svea**
**21109 Hamburg (DE)**

(74) Vertreter: **Beiersdorf AG**
**Patentabteilung**
**Unnastraße 48**
**20245 Hamburg (DE)**

(56) Entgegenhaltungen:
EP-A1- 2 949 312        WO-A1-2005/044213
WO-A1-2009/144139    WO-A2-2010/046011
FR-A1- 2 925 300        US-A1- 2005 196 365

• **David R. Karsa ET AL: "Surfactants Applications Directory" In: "Surfactants Applications Directory", 6. Dezember 2012 (2012-12-06), Springer Science & Business Media, XP055608755, ISBN: 978-94-010-5351-8 DOI: 10.1007/978-94-011-3038-7, das ganze Dokument**
• **DATABASE GNPD [Online] MINTEL; 21. Februar 2018 (2018-02-21), anonymous: "Exfoliating Cleansing Balm", XP055609366, gefunden im www.gnpd.com Database accession no. 5427159**

**Beschreibung**

Die Erfindung ist eine topisch applizierbare Zubereitung, die auf feuchter oder nasser Haut aufgebracht und verteilt werden kann. Die Nässe/Feuchtigkeit wird dabei aufgenommen und direkt in Hautpflege verwandelt. Vorteilhaft muss die Haut dann nicht abgetrocknet werden.

Die wasserfreie Zubereitung umfasst ein oder mehrere Lipide, mindestens einen W/O Emulgator und mindestens 0,05 Gew.% eines oder mehrerer Superabsorber.

In der täglichen Hautpflege und routinemäßigen Handlungen ist die menschliche Haut regelmäßig naß oder feucht. Sei es nach dem Waschen, Duschen, Baden, Schwimmen oder durch Schweiß und bedingt dadurch ein häufiges Abtrocknen der Haut.

Der Konsument möchte einerseits das ständige Abtrocknen vermeiden bzw. vermindern, da dies auch zur Austrocknung der Haut führt, und andererseits möchte er die Haut auch pflegen.

In der üblichen Routine wird nach der Hautreinigung diese getrocknet und anschließend ein Hautpflegeprodukt aufgetragen. Sind wir nass/feucht vom Schweiß, wischen wir den Schweiß ab und applizieren erst dann ein Hautpflegeprodukt, zum Beispiel ein Lichtschutzprodukt.

Diese Umstände zu vermeiden bzw. die dazu nötige Zeit zu verringern wäre wünschenswert.

Bekannt ist, dass die Mehrzahl der Hautpflegeprodukte Wasser enthalten. Dies gestaltet die Lagerung und den Transport von Hautpflegeprodukten schwierig.

Es gibt daher sog. Instant-Kosmetika, wasserfreie Kosmetik, die ohne Wasser angewendet werden können oder zuvor mit Wasser an zumischen sind.

Es gibt mittlerweile auch viele Kosmetik-Produkte, die aufgrund der Regelung bei Flugreisen, keine Flüssigkeiten über 100ml im Handgepäck mitnehmen zu dürfen, ohne Wasser auskommen.

Wasserlose Kosmetika haben zudem den Vorteil, dass die Produkte weniger bzw. keine Konservierungsstoffe benötigen. Auch bei der Verpackung ist Kosmetik ohne Wasser im Vorteil, weil sie meist weniger Material benötigt.

Wünschenswert ist es daher ein Produkt bereit zu stellen, welches die dargestellten Vorteile aufzeigt und die Nachteile vermeidet.

In der EP 2023888 A2 werden kosmetische wasserhaltige Zubereitungen offenbart, die Superabsorber auf Stärke Basis enthalten.

In der FR 2999919 werden O/W- und W/O-Emulsionen mit Superabsorber und Siloxanelastomeren beschrieben.

In der US 2013338052 werden wasserhaltige Formeln mit superabsorbierenden Partikeln beschrieben.

Weiterer Stand der Technik sind sogenannten In-Shower Produkte, die auf nasser Haut aufgetragen werden können. EP 2819639 A2 beschreibt solche Produkte.

Nach dem Auftragen der In-shower Produkte auf der nassen Haut wird die Haut abgespült und man muss sich weiterhin abtrocknen.

Eine für den Verbraucher sehr wesentliche, dabei aber nur schwer quantitativ messbare Eigenschaft kosmetischer Produkte ist ihre Textur und Sensorik. Unter dem Begriff "Textur" werden diejenigen Eigenschaften eines Kosmetikums verstanden, die auf den Gefügebau der Zubereitung zurückgehen, unter anderem durch Tast- und Berührungssinne empfunden und ggf. in mechanischen oder rheologischen Fließeigenschaften ausgedrückt werden können. Die Textur kann insbesondere mittels Sensorik getestet werden. Die gegebenenfalls mit Hilfe von Zusatzstoffen beeinflussbare Textur kosmetischer Produkte ist für den Verbraucher von nahezu gleicher Bedeutung wie deren objektiv feststellbaren Wirkungen.

Mit dem Begriff "Sensorik" wird die wissenschaftliche Disziplin bezeichnet, die sich mit der Bewertung von kosmetischen Zubereitungen auf Grund von Sinneseindrücken befasst. Die sensorische Beurteilung eines Kosmetikums erfolgt anhand der visuellen, olfaktorischen und haptischen Eindrücke.

- Visuelle Eindrücke: alle mit dem Auge wahrnehmbaren Merkmale (Farbe, Form, Struktur).

- Olfaktorische Eindrücke: alle beim Einziehen von Luft durch die Nase wahrnehmbaren Geruchseindrücke, die häufig in Anfangsgeruch (Kopfnote), Hauptgeruch (Mittelnote, Körper) und Nachgeruch (Ausklang) differenziert werden können. Auch die erst bei der Anwendung freigesetzten flüchtigen Stoffe tragen zum olfaktorischen Eindruck bei.

- Haptische Eindrücke: alle Empfindungen des Tastsinns, die vornehmlich Gefüge und Konsistenz des Produktes betreffen.

Die sensorische Analyse macht von der Möglichkeit Gebrauch, den sensorischen Gesamteindruck eines Produktes integral zu erfassen. Nachteile der sensorische Analyse sind die Subjektivität des Eindrucks, eine leichte Beeinflussbarkeit der Prüfpersonen und die dadurch bedingte starke Streuung der Ergebnisse. Diesen Schwächen begegnet man heute durch den Einsatz von Gruppen geschulter Prüfpersonen, gegenseitige Abschirmung der Prüfer sowie statistische Auswertung der meist zahlreichen Analysendaten.

Eine Aufgabe der vorliegenden Erfindung ist es daher, Zubereitungen zu Verfügung zu stellen, welche neben den für Kosmetika üblichen Kriterien wie Verträglichkeit, Lagerstabilität und dergleichen auch für den Verbraucher bisher nicht gekannte kosmetische, insbesondere sensorische, Leistungen bieten.

Überraschenderweise werden die dargestellten Probleme und Aufgaben gelöst durch die erfindungsgemäßen Zubereitungen sowie deren Verwendungen.

Die Erfindung umfasst in ihrer ersten Ausführungsform eine wasserfreie topisch applizerbare Zubereitung umfassend

    a) ein oder mehrere Lipide
    b) mindestens einen W/O Emulgator und
    c) mindestens 0,05 Gew.% eines oder mehrerer Superabsorber, bezogen auf die Gesamtmasse der Zubereitung.

Topisch applizierbare Zubereitungen sind kosmetische als auch dermatologische Zubereitungen, wie beispielsweise Gele, Salben, Creme, Lotion aber auch Zubereitungen in Form von Aerosolen oder Pumpsprays sowie Tränkungsmedien für eine Tuch-, Pad- oder Pflasterapplikation. Die Zubereitung kann flüssig, halbfest oder fest sein.

Die wasserfreie Zubereitung umfasst als bevorzugte Ausführungsform lediglich ein oder mehrere Lipide, mindestens einen W/O Emulgator und mindestens 0,05 Gew.% eines oder mehrerer Superabsorber.

In der einfachsten Ausführungsform umfasst die Zubereitung nur ein Lipid, ein W/O-Emulgator und ein superabsorbierendes Polymer.

Bevorzugt, vorteilhaft und sensorisch angenehmer ist eine Ausführungsform, in der die Zubereitung eine Kombination aus mindestens zwei Ölen, zwei W/O-Emulgatoren und einem Superabsorber umfasst.

Vorteilhaft und äußerst erstaunlich dabei ist, dass die Wasseraufnahme in wenigen Sekunden und damit das Hautabtrocknen in kurzer Zeit erfolgt.

Es wurde mit der erfindungsgemäßen Zubereitung eine neue Technologie der zweckdienlichen Hautpflege erfunden, die sowohl Annehmlichkeiten als auch Nutzen für den Verbraucher bei der täglichen Körperpflege ermöglicht.

Die erfindungsgemäße Zubereitung ermöglicht das zeitgleiche Abtrocknen und die Hautpflege nach dem Waschen, Baden oder Duschen in einem Schritt.

Man kann die erfindungsgemäße Zubereitung auf die noch nasse oder feuchte Haut auftragen, verteilen und im Anschluss befindet sich die Haut in dem gleichen Zustand, als hätte man sich abgetrocknet, mit z.B. einem Handtuch, und anschließend erst ein Hautpflegeprodukt aufgetragen.

Bei der Anwendung der erfindungsgemäßen Zubereitung ist das Wasser, die Nässe, Feuchtigkeit auf der Haut nach dem Waschen, Duschen, Baden oder Schwitzen sogleich in eine Haut-, und Körperpflege umgewandelt worden.

Die Vorteile der neuen Technologie sind vielseitig und nachstehend werden einige Beispiele genannt:

- die Benutzung eines Handtuches nach dem Waschen, Baden, Duschen ist entbehrlich
- die Häufigkeit des Waschens von Handtüchern reduziert sich
- Zeitersparnis, während des Trocknens wird zugleich die Haut eingecremt; es wird das Wasser, die Feuchtigkeit auf der Haut genutzt
- Reduktion der Produktgröße und -volumen; das normalerweise in Kosmetika enthaltene Wasser entfällt. Es kann eine Art Konzentrat genutzt und transportiert werden. Es werden kleinere Behältnisse benötigt und damit weniger Müll produziert. Beim Transport wird pro Verbrauchseinheit weniger Kraftstoff verbrannt.
- Individualisierung des Badens/Dusche bzw. der Hautpflege. Man/Frau kann selber steuern, was für ein Produkt auf der Haut aufgetragen wird, indem man mehr oder weniger Wasser auf der Haut belässt. Es entstehen keinerlei Nachteile auch wenn das erfindungsgemäße Produkt auf trockner Haut angewendet wird. Es fühlt sich dann reichhaltiger an, weil es nicht mit Wasser verdünnt wird.
- hohe Hautbefeuchtungsleistung
- die erfindungsgemäße Zubereitung eröffnet multiple Vorteile in der Galenik, denn aufgrund der Wasserfreiheit können beispielsweise wasserempfindliche Stoffe einfacher eingearbeitet werden
- auf Konservierungsmittel kann verzichtet werden
- gereizte Haut muss nicht mehr mit einem Handtuch unangenehm abgerubbelt und wird nicht noch weiter gereizt
- man kann auf diese Weise sogar UV-Schutz auf nasser Haut auftragen
- man kann auf diese Weise sogar UV-Schutz auf nasser Haut auftragen, selbst wenn man sich noch einmal abduschen und abtrocknen möchte, sog. In-Shower Anwendung
- man kann auf diese Weise sogar wasserfesten UV-Schutz selbst ohne Filmbildner anbieten
- am Strand muss man sich vor der Produktapplikation (UV Schutz oder auch After Sun) nicht mit einem sandigen Handtuch abtrocknen

Gegenüber den Zubereitungen des Standes der Technik, die Superabsorber umfassen, wie beispielsweise FR 2999919, handelt es sich bei den erfindungsgemäßen Zubereitungen vorteilhaft um keine Emulsionen und zudem sind die erfindungsgemäßen Zubereitungen wasserfrei.

Die erfindungsgemäßen als auch optional möglichen Bestandteile der topisch applizierbaren Zubereitung werden bevorzugt gewählt aus den nachstehend detailliert dargestellten Gruppen:

a) Lipide

Im Rahmen der vorliegenden Offenbarung wird als Oberbegriff für Fette, Öle, Wachse und dergleichen der Ausdruck "Lipide" verwendet, wie dem Fachmanne durchaus geläufig ist. Auch werden die Begriffe "Ölphase" und "Lipidphase" synonym angewandt.

Die erfindungsgemäße Zubereitung umfasst ein oder mehrere Lipide.

Bevorzugt sind Lipide mit einer geringen Viskosität und hohem Spreitwert.

Die Viskosität (25°C, dynamische) der Lipide beträgt bevorzugt unter 250 mPas und der Spreitwert beträgt vorteilhaft über 400.

Zur Messung der Viskosität kosmetischer Zubereitungen stehen u.a. Rotationsrheometer zur Verfügung.

Die Messung der Viskosität mit einem Rotationsrheometer erfolgt indem ein Messkörper in der zu vermessenden Substanz rotiert. Die Viskosität der Substanz verhält sich proportional zu der Kraft, mit der sie der Rotation des Messkörpers entgegenwirkt. So kann die Viskosität mit Hilfe eines Viscotester VT-02, der Firma Haake bei 25°C mit Hilfe des Drehkörpers 1 bzw. 2 und Ablesung der Skala 1 bzw. 2 bestimmt werden.

Die Messung des Spreitwertes erfolgt folgendermaßen:

Die Lipide werden bei Raumtemperatur vermessen. Eine Messung dauert pro Filterpapier 10 Minuten. Als Filterpapier kann rundes Filterpapier Whatman 589/2 110mm ashless/white ribbon verwendet werden. 25 $\mu$l des zu vermessenden Lipides werden zentral von einer Höhe von ca.

30 mm auf das Filterpapier appliziert, genauer gesagt, pippetiert. Es ist darauf zu achten, dass das Lipid bei der Applikation nicht verstreut aufgetragen wird. Mit der Applikation wird die Stoppuhr gestartet.

Nach 10 Minuten Spreitzeit wird der entstandene Fleck umrandet. Diese Messung wird auf fünf Filterpapieren für ein Lipid durchgeführt. Vom umrandeten Fleck wird an drei verschiedenen Stellen der Durchmesser in mm ermittelt. Unter Verwendung von 15 Werten (5 Filterpapiere mit jeweils 3 Durchmessern) wird der Durchschnittswert ermittelt. Mit diesem Durchschnittswert wird dann die Fläche des gespreiteten Lipids berechnet (Flächenberechnung eines Kreises):

$$A = \pi * r^2 \text{ oder } A = \pi * (d/2)^2$$

Der Spreitwert wird in der Einheit $mm^2/(10\text{ min})$ für $25\mu L$ angegeben

Vorteilhaft sind daher Lipide zu wählen aus folgender Tabelle:

| INCI | Viskosität [mPas] | Spreitwert [mm²/10 min] |
|---|---|---|
| Dibutyl Adipate | 5 | 935 |
| Isopropyl Myristate | 4,6 | 707 |
| Isodecyl Neopentanoate | 3,8 | 962 |
| Octyldodecanol | 47,7 | 440 |
| Caprylic/Capric Triglyceride | 20 | 570 |
| Propylene Glycol Dicaprylate/Dicaprate | 12 | 868 |
| Dicaprylyl Ether | 3,3 | 1020 |
| Paraffinum Liquidum | 27,4 | 550 |
| Isopropyl Palmitate | 6,2 | 625 |
| C13-16 Isoparaffin | 2,6 | 1018 |
| Isohexadecane | 3,7 | 990 |
| Cyclomethicone | 5 | 804 |
| Hydrogenated Polydecene | 45,6 | 510 |

(fortgesetzt)

| INCI | Viskosität [mPas] | Spreitwert [mm$^2$/10 min] |
|---|---|---|
| Octyldodecyl Myristate | 24,2 | 755 |
| Decyl Oleate | 14,1 | 668 |
| Butylene Glycol Dicaprylate/Dicaprate | 9,6 | 813 |
| Dicaprylyl Carbonate | 6,3 | 875 |
| Hydrogenated Polyisobutene | 34,1 | 616 |
| Isopropyl Isostearate | 9,5 | 790 |
| Cetearyl Isononanoate | 14,9 | 780 |
| Isopropyl Stearate | 6,8 | 910 |
| Phenyl Trimethicone | 17,3 | 631 |
| C12-15 Alkyl Benzoate | 12,2 | 730 |
| Ethylhexyl Cocoate | 7,9 | 930 |
| Squalane | 30 | 594 |
| Ethylhexyl Stearate | 29 | 697 |
| Cetearyl Ethylhexanoate+Isopropyl Myristate | 24 | 683 |
| C12-13 Alkyl Lactate | 17 | 800 |
| Coco-Caprylate/Caprate | 5 | 755 |
| Persea Gratissima Oil | 180 | 416 |
| Simmondsia Chinensis Seed Oil | 35,8 | 449 |

Der Anteil an einem oder mehreren Lipiden wird vorteilhaft im Bereich von 25 Gew.% bis 65 Gew.%, bevorzugt im Bereich von 35 Gew.% bis 55 Gew.%, besonders bevorzugt im Bereich von 30 Gew.% bis 50 Gew.% gewählt, bezogen auf die Gesamtmasse der Zubereitung.

Handelt es sich um eine Lichtschutzzubereitung, die entsprechende UV-Filter umfasst, die wiederum auch als Lipide verstanden werden können, so liegt der Anteil an Lipiden inklusiver eventueller UV-Filter im Bereich von 20 Gew.% bis 75 Gew.%, bevorzugt im Bereich 35 Gew.% bis 65 Gew.%, besonders bevorzugt im Bereich von 40 Gew.% bis 60 Gew.%, bezogen auf die Gesamtmasse der Zubereitung.

In einer weiteren Ausführungsform sind vorteilhaft als Lipide Wachse zu wählen. Weiter vorteilhaft sind neben Wachsen weitere Lipide enthalten.

Als Wachse können erfindungsgemäß auch Fette und fettähnlichen Substanzen mit wachsartiger Konsistenz eingesetzt werden. Hierzu gehören u.a. Fette (Triglyceride), Mono und Diglyceride, natürliche und synthetische Wachse, silikonbasierte Wachse, Kohlenwasserstoffwachse, Fett-und Wachsalkohole, Ester von Fettalkoholen und Fettsäuren sowie Fettsäureamide oder beliebige Gemische dieser Substanz.

Besonders bevorzugt werden die Wachse aus der Gruppe der Fette, insbesondere aus der Gruppe natürliche Wachse gewählt, wie insbesondere Shorea Stenoptera Seed Butter, Hydrogenated Vegetable Oil, Hydrogenated Coco-Glycerides, Butyrospermum Parkii Butter, Theobroma Cacao (Cocoa) Seed Butter, Mango Butter, Hydrogenated Palm Kernel Glycerides, Hydrogenated Palm Glycerides, Sunflower Seed Wax, Soybean Glycerides, Butyrospermum Parkii Unsaponifiables, Hydrogenated Castor Oil, Wollwachs, Cera Alba, Beeswax, Zuckerrohrwachs, Cera Carnauba, Candelillawachs,Japanwachs, Hydrogenated Rapeseed Oil, Shellac Wax, Hydrogenated Lecithin, Hydrogenated Soybean Oil.

Aus der Gruppe der synthetischen Wachse werden insbesondere ausgewählt Cera Microcristallina, Synthetic Beeswax, Synthetic Wax, Polyethylene, Paraffin Wax, Ceresin, Ozokerite.

Aus der Gruppe der Fettsäuren werden insbesondere ausgewählt Palmitic Acid, Stearic Acid.

Aus der Gruppe der Ester aus Fettsäuren werden insbesondere ausgewählt Cetearyl Nonanoate, Methyl Palmitate, Glyceryl Tribehenate, Glyceryl Laurate, Glyceryl Stearate, Cetyl Palmitate; Shea Butter Oleyl Esters, PEG-8 Beeswax.

Als Fettalkohole werden bevorzugt C14 bis C22 Fettalkohole verwendet. Bevorzugt werden die Fettalkohole gewählt aus der Gruppe linearen Fettalkohole, insbesondere Myristylalkohol ($C_{14}H_{30}O$), Cetylalkohol (oder Palmitylalkohol) ($C_{16}H_{34}O$), Stearylalkohol (oder Octadecylalkohol) ($C_{18}H_{38}O$) sowie Cetylstearylalkohol (Cetearylalkohol),Behenylalkohol, Lanolin Alcohol, ein Gemisch der Alkohole Cetylalkohol (Hexadecanol) und Stearylalkohol (Octadecanol).

Erfindungsgemäß bevorzugt werden Wachse gewählt, die einen Schmelzpunkt im Bereich zwischen 30 - 120 ° aufweisen. Besonders bevorzugt sind Wachse mit einem Schmelzpunkt im Bereich 40°C bis 100°C, besonders bevorzugt im Bereich 40°C bis 90°C.

Der bevorzugte Schmelzpunkt ergibt sich zum einen aus der Körpertemperatur, da die Lipide da noch nicht schmelzen sollten (Untergrenze) und gleichzeitig noch gut verarbeitbar sein (Obergrenze).

Die bevorzugt genannten Wachse beeinflußen die Sensorik der erfindungsgemäßen Zubereitung positiv.

"Wachsig" fühlt sich im Gegensatz zu "ölig" eher trocken und stumpf an und führt daher zu einem attraktiveren Produkt.

Der Anteil an einem oder mehreren Wachsen wird vorteilhaft gewählt im Bereich von 1 Gew.% bis 20 Gew.%, bevorzugt 2% -12 Gew.%, besonders bevorzugt 4 Gew.% bis 10 Gew.%, bezogen auf die Gesamtmasse der Zubereitung.

## b) W/O Emulgatoren

Die erfindungsgemäße Zubereitung umfasst ein oder mehrere Wasser in Öl Emulgatoren.

Erfindungsgemäße W/O-Emulgatoren binden das Wasser in der Ölphase und unterstützen so die Trocknungseigenschaften.

Im Vergleich dazu zeigen O/W-Emulgatoren, dass das Wasser in der äußeren Phase angelagert wird und das Hautgefühl bleibt ein nasses, feuchtes Gefühl, so dass der erfindungsgemäße Zweck nicht erfüllt wird.

In einer W/O/W - Emulsion wurde ebenfalls der erfindungsgemäße Trocknungseffekt nicht erzielt.

Bevorzugt sind zwei W/O-Emulgatoren zu wählen, da somit eine noch bessere Sensorik und vor allem schnellere Trocknungskinetik erreicht wird. Des Weiteren läßt sich so die Optik während des Verteilens steuern.

Zu den W/O-Emulgatoren zählt erfindungsgemäß auch die Gruppe der W/Si-Emulgatoren.

Im Allgemeinen gelten Emulgatoren mit einem HLB-Wert bis ca. 8 als W/O-Emulgatoren. O/W-Emulgatoren hingegen weisen HLB-Werte von größer 8 bis 15 auf. Substanzen mit HLB-Werten größer 15 werden häufig als Lösungsvermittler bezeichnet. Im Stand der Technik werden W/O-Emulsionen zuweilen durch Zusatz von O/W-Emulgatoren (HLB > 8) stabilisiert, die die Funktion eines Co-Emulgators ausüben. Der Verzicht auf solche Substanzen verringert die Rohstoffkomplexität und vermeidet ein Umschlagen der Formulierung in den nicht gewünschten O/W-Bereich. Es ist erfindungsgemäß vorteilhaft auf den Zusatz von Emulgatoren mit einem HLB > 8 zu verzichten.

Als Hauptemulgator dient in der erfindungsgemäßen Zubereitung ein W/O-Emulgator, der aus der Liste der bekannten W/O-Emulgatoren zu wählen ist. Bevorzugt weisen der oder die W/O-Emulgatoren einen HLB-Wert von kleiner gleich 8 auf.

Ein oder mehrere vorteilhaft einsetzbare W/O-Emulgatoren sind aus der Gruppe Polyglyceryl-n Diisostearate, Polyglyceryl-n-dipolyhydroxystearate, insbesondere Polyglyceryl-n Dipolyhydroxystearate, Diisostearoyl Polyglyceryl-n Dimer Dilinoleate und/oder Polyglyceryl-n Diisostearate/Polyhydroxystearate/Sebacate zu wählen

Ein oder mehrere vorteilhafte W/O-Emulgatoren sind aus der Gruppe Polyglyceryl-3 Diisostearate, Polyglyceryl-2 Dipolyhydroxystearate, Diisostearoyl Polyglyceryl-3 Dimer Dilinoleate und/oder Polyglyceryl-4 Diisostearate/Polyhydroxystearate/Sebacate zu wählen.

Der Anteil an der Gesamtmenge an W/O-Emulgatoren beträgt bevorzugt 0,25 bis 10 Gew.%, insbesondere 0,75 bis 7 Gew.%, bezogen auf die Gesamtmasse der Zubereitung.

Bevorzugt ist die Kombination aus 5 Gew.% Polygylceryl-3 Diisostearate und 2 Gew.% Polyglyceryl-2 Dipolyhydroxystearate.

Erfindungsgemäß besonders bevorzugt ist die Kombination aus Diisostearoyl Polyglyceryl-3 Dimer Dilinoleate und Polyglyceryl-4 Diisostearate/Polyhydroxystearate/Sebacate im Verhälnis 2:1 bis 1:2, besonders bevorzugt ist das Verhältnis 1:1.

Erfindungsgemäß besonders bevorzugt ist die Kombination aus 0,25 - 1,5% Gew. Diisostearoyl Polyglyceryl-3 Dimer Dilinoleate und 0,25 -1,5%Gew. Polyglyceryl-4 Diisostearate/Polyhydroxystearate/Sebacate.

Die bevorzugt genannten Emulgatoren erzielen gute Ergebnisse bezüglich Trocknungsgefühl, Sensorik (Leichtigkeit) und Preis.

Bevorzugt wird der Anteil an Polyglyceryl-3 Diisostearate im Bereich von 2 bis 7 Gew.%, insbesondere 5 Gew.%, der Anteil an Polyglyceryl-2 Dipolyhydroxystearate im Bereich von 1 bis 3 Gew.%, insbesondere 2 Gew.%, der Anteil an Diisostearoyl Polyglyceryl-3 Dimer Dilinoleate im Bereich von 0,5 bis 2 Gew.%, insbesondere 1 Gew.%, und der Anteil an Polyglyceryl-4 Diisostearate/Polyhydroxystearate/Sebacate im Bereich 0,5 bis 2,5 Gew.%, insbesondere 1 Gew.%, gewählt, jeweils bezogen auf die Gesamtmasse der Zubereitung.

Das System 1 Gew.% Diisostearoyl Polyglyceryl-3 Dimer Dilinoleate und 1 Gew.% Polyglyceryl-4 Diisostearate/Polyhydroxystearate/Sebacate ist besonders bevorzugt zu wählen. Im Falle einer UV Filter haltigen Formel ist das System 0.5 Gew.% Diisostearoyl Polyglyceryl-3 Dimer Dilinoleate und 0.5 Gew.% Polyglyceryl-4 Diisostearate/Polyhydroxystearate/Sebacate ist besonders bevorzugt zu wählen

Die Gewichtsanteile beziehen sich jeweils auf die Gesamtmasse der Zubereitung.

c) Superabsorber

Die erfindungsgemäße Zubereitung umfasst mindestens 0,05 Gew.% eines oder mehrerer Superabsorber, bezogen auf die Gesamtmasse der Zubereitung.

Für Superabsorber sind auch Bezeichnungen wie "hochquellfähiges Polymer", "Hydrogel", "Hydrogel bildendes Polymer", "Wasser absorbierendes Polymer", "absorbierendes gelbildendes Material", "quellfähiges Harz", "wasserabsorbierendes Harz" "Gelierungsmittel" oder ähnliche Bezeichnungen gebräuchlich. Es handelt sich dabei vornehmlich um vernetzte hydrophile Polymere, insbesondere Polymere aus (co)polymerisierten hydrophilen Monomeren, Pfropf(co)polymere von einem oder mehreren hydrophilen Monomeren auf einer geeigneten Pfropfgrund- lage, vernetzte natürliche Kohlenhydrate zum Beispiel Cellulose- oder Stärkeether, vernetzte Carboxymethylcellulose und Carboxymethylstärken (Natriumstärkeglycolate bzw. Natriumcelluloseglycolate), teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure am weitesten verbreitet sind. Superabsorber sind Materialien, die ein Vielfaches ihrer Masse an Flüssigkeiten aufnehmen können. Wenn auch die Oberfläche der Superabsorber quervernetzt ist kann sogar eine gewissen Retention erhalten werden, dass heißt, dass sie auch bei mechanischer Beanspruchung aufgenommene Flüssigkeiten nicht wieder abgeben. Solche Flüssigkeiten können Wasser oder wässrige Lösungen und organische Flüssigkeiten und deren Mischungen sein. Im Falle von wässrigen Lösungen bestehen die Superabsorber insbesondere aus vernetzten Makromolekülen mit ionischen und/oder polaren Gruppen. Seit dem Beginn der siebziger Jahre wurden auf der Basis von neutralisierter, vernetzter Polyacrylsäure (PANV) leistungsfähige und vielseitig einsetzbare Superabsorber entwickelt.

Unter Superabsorbern (SA) werden vorzugsweise Polymere mit folgender chemischer Zusammensetzung verstanden:

vollsynthetisch:

- vernetzte Polyacrylate und deren Derivate (zum Beispiel Methacrylate)

- Polyvinylacetat-Acrylsäure-Copolymere

- Polyvinylalkohol-Copolymere

- Polyethylenoxid

- Isobutylen-Maleinsäure-Copolymere

- hydrolysiertes Polyacrylnitril

halbsynthetisch:

- Stärke-Polyacrylat-Pfropfcopolymere

- Hydrolysierte Stärke-Polyacrylnitril- Pfropfcopolymere

- Carboxymethylcellulose

- Carboxymethylstärken (auch bekannt als starch glycolates)

natürliche:

- Alginate

Das mit weitem Abstand wichtigste Einsatzgebiet von Superabsorbern ist das Absorbieren von Körperflüssigkeiten. Superabsorber werden beispielsweise in Windeln für Kleinkinder, Inkontinenzprodukten für Erwachsene, Damenhygieneprodukten oder in der Wundversorgung verwendet. Andere Anwendungsgebiete sind im landwirtschaftlichen Gartenbau, als Wasserspeicher zum Schutz vor Feuer, zur Flüssigkeitsabsorption in Lebensmittelverpackungen oder ganz allgemein zur Absorption von Feuchtigkeit.

In der Kosmetik ist der Einsatz von Superabsorbern weitgehend selten zu beobachten. Lediglich sind Produkte bekannt in denen durch den Einsatz von Polyacrylat-Superabsorbern eine "sorbetartige" Oberfläche erzeugt werden kann.

Polare Polymere wie zum Beispiel Polyacrylsäure binden schon große Mengen des ebenfalls polaren Wassers (1000fache Masse) in ihrem Inneren und quellen in diesem Prozess.

Durch die Natriumionen innerhalb der Polymerstruktur bildet das Molekül zusätzlich einen starken Salzcharakter aus. Es kommt aufgrund eines osmotischen Drucks zur weiteren Absorption von Flüssigkeit.

Dieser osmotische Druck bedingt letztlich die Aufnahmekapazität des Superabsorbers.

Daraus ergeben sich folgende Zusammenhänge: Je mehr Natriumionen im Polymer eingebaut sind, desto größere Mengen Flüssigkeit können aufgenommen werden. Je höher die Konzentration an Salzen in der Flüssigkeit, desto geringer wird die Absorption ausfallen. Dies erklärt, dass reines Wasser in Größenordnungen um das mehrere Hundertfache des Superabsorbereigengewichts absorbiert wird, wohingegen Salzlösungen, wie Urin etc., die Kapazität des Superabsorbers deutlich begrenzen. Deswegen wird von einer physiologischen Kochsalzlösung nur das 100fache aufgenommen.

Das beschriebene Prinzip funktioniert nicht nur mit Natrium sondern auch mit anderen Kationen.

Aus der Funktionsweise leitet sich auch ab, weshalb unpolare Flüssigkeiten wie Öle nicht absorbiert werden können.

Aus der Gruppe der vollsynthetischen Superabsorber sind erfindungsgemäß bevorzugt zu wählen:

- Vernetzte Polyacrylate, insbesondere Natriumpolyacrylat- crosspolymer-2 (Aquakeep 10SH-NFC)
- Vernetzte Polyacrylamide
- Vernetzte Polymethacrylate
- Polyvinylether
- Polyvinylpyridine
- Polyvinylmorpholinone,
- N,N-dimethylaminoethyl oder N,N-diethylaminopropyl acrylates und Methacrylates
- Poloxamer
- Polyvinylalkohol
- Polyvinyl alcohol-polyethylene glycol graft copolymer
- Maleic acid anhydride/isobutene copolymer
- Polyvinylpolypyrrolidone
- Poly(vinyl glyoxylic acid)
- Acryloacrylamide/natriumacrylate copolymer
- Starch/Acrylamide/Sodium acrylate copolymer

Aus der Gruppe der Superabsorber basierend auf Polysacchariden sind erfindungsgemäß bevorzugt zu wählen:

- Natriumpolyacrylate starch
- Alginate
- Alginate esters
- Hydroxyethylcellulose
- Hydroxypropyl cellulose
- Hydroxypropyl methyl cellulose
- Carboxyalkyl cellulose
- Natriumcarboxymethyl cellulose
- Croscarmellose sodium
- Natrium starch glycolate (Glycolys, Explotab, Vivastar, Primojel)
- Glucomannane
- Galactomannane (z.B. von Konjac)
- Xyloglucan
- (modified) Cellulose fibers
- Cotton fibers
- (modified) Starches
- Xanthan gum
- Cellulose gum
- Carrageenan gum

- Guar gum
- Arabic
- Tragacanth
- Pustulan
- Callose
- Laminarin
- Guluronic acid
- Pullula lichenin
- Amylose
- Amylopectin
- Dextran
- Pectin
- Inulin
- Chitin
- Hyaluronic acid

Aus der Gruppe der Superabsorber basierend auf Aminosäuren sind erfindungsgemäß bevorzugt zu wählen:

- Polyaspartic acid
- Polyglutamic acid
- Poly($\varepsilon$-L-lysine)
- Gelatine
  sowie
- Phytocolloide

Vorteilhaft ist der Einsatz von Superabsorbern aus nachwachsenden Rohstoffen, da sie gesundheitlich unbedenklicher und umweltverträglicher und zudem hautverträglicher erscheinen.

Als Basis für die Herstellung von Superabsorbern aus nachwachsenden Rohstoffen sind (Poly)Sacharide wie Cellulose, Stärke, Chitin und Pektine geeignet.

Bevorzugt sind Superabsorber, welche auf Basis natürlich Kohlehydrate aufgebaut sind, ganz besonders bevorzugt Glycolate, auch bekannt als Carboxymethyl derivatisierte Polymere, bevorzugt Biopolymere.

Die Superabsorber auf Basis von Kohlehydraten sind deswegen zu bevorzugen, da sie überraschenderweise häufige Nachteile wie Klumpen- oder Graupenbildung auf der Haut nicht zeigen.

Bevorzugt sind insbesondere Carboxymethylcellulose und Carboxymethylstärken (Natriumstärkeglycolate bzw. Natriumcelluloseglycolate).

Die erfindungsgemäß bevorzugte vernetzte Carboxymethylstärke weist idealerweise eine bevorzugte Teilchengrößenverteilung auf in dem mindestens 95% der Teilchen kleiner als 130 $\mu$m, bevorzugt kleiner als 120 $\mu$m sind. Die mittlere Partikelgröße liegt zwischen 30-70 $\mu$m.

Erfindungsgemäß bevorzugte Superabsorber sind quervernetzte Carboxymethylstärken (Sodium starch glycolate) charakterisiert dadurch, dass sie ein Vielfaches ihres Gewichts an Wasser aufnehmen können, zum Beispiel Explotab and Vivastarch (z.B. Explotab 3500, Explotab CLV etc). Die Wasseraufnahmekapazität ist demnach ein Kriterium für bevorzugt zu wählende Superabsorber.

Quervernetzung ist ein Prozess in dem Polymere untereinander verknüpft, verlinkt, verbunden werden und somit ein dreidimensionales Netzwerk bilden. Der Prozess kann direkt bei der Synthese des Polymers stattfinden oder auch nachträglich.

Die Quervernetzung kann einen Einfluss auf die Eigenschaften der quervernetzen Polymere haben, wie zum Beispiel das Wasseraufnahmevermögen und auch die Wasserretention.

Auch die Tatsache, ob während der Synthese des Polymers oder danach quervernetzt wurde, kann die Eigenschaften des finalen SA beeinflussen.

Erfindungsgemäß sind ein oder mehrere wasseraufnehmende Stoffe in der Zubereitung enthalten, die jeweils ein Vielfaches ihres Gewichts an Wasser aufnehmen können.

Superabsorber können dabei erfindungsgemäß bevorzugt das bis zu 1000-fache ihres Gewichts, bevorzugt bis zu 200-fache ihres Gewichts an Flüssigkeit aufnehmen. Besonders bevorzugt sind Superabsorber mit einer Flüssigkeitsaufnahmekapazität, insbesondere Wasseraufnahmekapazität, im Bereich vom 50-fachen ihres Gewichtes bis zu dem 5-fachen ihres Gewichtes.

Bevorzugt wird ein Superabsorber eingesetzt. Es kann jedoch je nach gewünschten Eigenschaften, wie zum Beispiel die Sensorik, notwendig sein mehrere Superabsorber zu kombinieren.

Der Anteil an einem oder mehreren erfindungsgemäßen Superabsorbern beträgt mindestens 0,05 Gew.%,

bezogen auf die Gesamtmasse der Zubereitung. Bevorzugt liegt der Anteil an einem oder mehreren Superabsorbern im Bereich von 0,05 bis 20 Gew.%, bevorzugt 0,1 bis 10 Gew.%, bezogen auf die Gesamtmasse der Zubereitung.

Erfindungsgemäß bevorzugt ausgeschlossen sind Sensorikadditive, wie vor allem um Aluminium Starch Octenylsuccinate, bei denen es sich nicht um Superabsorber handelt.

Erstaunlicherweise zeigen die Superabsorber und hier insbesondere die bevorzugt zu wählenden Absorber keinen negativen Einfluss auf die Hautbefeuchtung.

Erwartungsgemäß wäre ein Feuchtigkeitsentzug auch der Haut durch Superabsorber denkbar gewesen. Dies zeigte sich erstaunlicherweise aber nicht.

Die Hautfeuchtigkeitsmessungen wurden durchgeführt nach dem Prinzip der Kapazitätsmessung der Haut als dielektrisches Medium.

Dazu wird ein Gerät Corneometer CM 825 von Courage + Khazaka (Köln, Deutschland) verwendet. Die Parameter sind die Permittivität [a. U.], die aus dem Median aus wiederholter Messung (10) erzeugt werden. Eine Erhöhung Permittivität zeigt dementsprechend eine verbesserte Hautbefeuchtung an.

Getestet wurden zwei Zubereitungen (652, 654), wobei die Zubereitung 654 nicht erfindungsgemäß ist, und keinen Superabsorber enthält.

| A_652 | B_654 | INCI/Gew.% |
|---|---|---|
| 7,21 | 7,21 | Isoamyl Laurate |
| 5 | 0 | Sodium starch glycolate |
| 3 | 3 | Hydrogenated Castor Oil |
| 22,25 | 22,25 | Isopropyl Palmitate |
| 2,37 | 2,37 | Octyldodecanol |
| 2,37 | 2,37 | C12-15 Alkyl Benzoate |
| 1 | 1 | Diisostearoyl Polyglyceryl-3 Dimer Dilinoleate |
| 1 | 1 | Polyglyceryl-4 Diisostearate/Polyhydroxystearate/Sebacate |
| 5 | 5 | Calcium Starch Octenylsuccinate + Aqua + Calcium Chloride |
| 10 | 10 | Dimethicone + Dimethicone Crosspolymer |
| 0,8 | 0,8 | Parfum |
| 6 | 6 | Glycerin + Aqua |
| 4 | 4 | Magnesium Stearate |
| 30 | 35 | Alcohol Denat. + Aqua |

Es gab keine signifikanten Unterschiede in der oben beschriebenen Messung zwischen den beiden in der Tabelle beschriebenen Formeln (A_652 und B_654). Somit kann gezeigt werden, dass die Anwesenheit eines Superabsorbers keinen negativen Einfluß auf die Hautbefeuchtung hat. Des Weiteren gab es keine Inkompatibilitäten oder Reaktion. Es wurden keine Beschwerden beobachtet.

d.) Wasserfrei

"Frei von" bedeutet, dass der Anteil an den zu vermeidenden Stoffen weniger als 1 Gew.% beträgt. Damit ist gewährleistet, dass geringe Mengen dieser Stoffe, die zufällig eingeschleppt werden oder rohstoffbedingte Zusätze darstellen, mit umfasst sind, da sie in diesen geringen Mengen keinen Einfluss ausüben können, insbesondere keinen Einfluss auf die erfindungsgemäßen Eigenschaften.

Die erfindungsgemäße Zubereitung ist wasserfrei. Dies bedeutet, dass die Zubereitung weiterhin als wasserfrei anzusehen ist auch wenn geringe Mengen Wasser enthalten sind, die eingeschleppt wurden oder Wasser, welches keinerlei Auswirkungen auf die Eigenschaft der Zubereitung hat.

Durch Bestandteile der Zubereitung, wie beispielsweise Hautbefeuchtungsmittel oder Ethanol, kann der Wassergehalt in der Zubereitung bis zu 1 Gew.% betragen, idealerweise bis zu 0,6 Gew.%, bzw. bevorzugt weniger als 0,3 Gew.%, insbesondere 0 Gew.%, bezogen auf die Gesamtmasse der Zubereitung, und gilt dann erfindungsgemäß dennoch als wasserfrei.

e) UV-Filter

Ein Problem des notwendigen Sonnenschutzes der Haut ist, dass das Sonnenschutzmittel auf der gesamten Haut verteilt werden sollte. Ein mehrfaches an- und auskleiden ist dazu eigentlich nötig. Die erfindungsgemäße Zubereitung bietet hier eine geniale Vereinfachung. Nach dem Duschen oder Baden wird auf die unbekleidete feuchte oder nasse Haut die erfindungsgemäße Zubereitung, die ein oder mehrere UV Filter umfasst, aufgetragen. Man muss sich nicht abtrocknen und kann direkt auftragen. Danach ist man trocken und zugleich mit Sonnenschutz versehen. Diese Verwendung ist auch am Strand mit Meerwasser möglich.

Weitere Vorteile sind:

Der Lichtschutz (SPF) ist, wenn das Produkt auf nasser Haut aufgetragen wird, vergleichbar mit dem Lichtschutz beim Auftrag auf trockener Haut (Iso 24444:2010 (E)).

Dabei ist es besonders überraschend, dass die erfindungsgemäße Zubereitung sich angenehm und unproblematisch auftragen läßt und ein SPF von mindestens 30 erreicht wird.

Eine weitere Überraschung ist, dass die erfindungsgemäße Zubereitung wasserfest (gemäß Wasserfestigkeit nach Colipa 2005) ist, obwohl keine Filmformer in der Zubereitung enthalten sind.

Selbst wenn man das Bedürfnis hat, sich einmal kurz nach Applikation abzuspülen, verbleibt weiterhin ein signifikanter SPF von mindestens 25, entsprechend einem Testdesign der der Iso 24444:2010 (E) angelehnt ist. Die Haut wird 1 Minute gewässert. Dann werden 2,0 (+/-0.05mg)/cm2 Zubereitung innerhalb von 30 Sekunden aufgetragen. Nach einer Wartezeit von 1,5 Minuten wird 30 Sekunden abgeduscht und trocken getupft. Im Anschluß daran wurde der SPF vermessen.

Es ist daher vorteilhaft im Sinne der vorliegenden Erfindung, wenn die Zubereitung einen oder mehrere UV-Filter enthält.

Ein oder mehrere UV-Filter werden vorteilhaft gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Der Anteil an ein oder mehreren UV-Filtern wird bevorzugt im Bereich von mehr als 0,1 Gew.%, , bis zu 50 Gew.% insbesondere im Bereich von 5 bis 35 Gew.% gewählt, bezogen auf die Gesamtmasse der Zubereitung.

Die erfindungsgemäßen Zubereitungen umfassend ein oder mehrere UV-Filter lassen sich auf nasser oder feuchter Haut applizieren und erzielen so einen UV Schutz.

Die Verwendung der Zubereitung umfassend ein oder mehrere UV-Filter ist damit geeignet zur Applikation auf nasser Haut, zur Trocknung der Haut und zeitgleichem Schutz der Haut vor UV Strahlung.

Alles erfolgt in einem Anwendungsschritt.

Auch nachfolgende Verfahrensschritte, wie Abspülen und Abtrocknen, führen zu einem Schutz der Haut vor UV Strahlung.

Überraschenderweise kann bei der Verwendung der Zubereitung zum Schutz der Haut vor UV Strahlung auf Filmbildnern verzichtet werden.

f) Stabilisierung, Viskositätsverbesserung

Nachteilig an verschiedenen kosmetischen oder dermatologischen Zubereitungen ist, dass diese über den Lagerungs- und Nutzungszeitraum eine gleichbleibende Qualität (optisch, olfaktorisch, mikrobiell etc.) haben müssen. Dazu zählt auch die physikalische Stabilität. Die physikalische Stabilität kann man oft durch Erhöhung der Viskosität und der damit verbundenen verringerten Diffusion der Öltröpfchen erreichen. Im Stand der Technik findet man hier viele Beispiele wie man zum Beispiel bei einer O/W Emulsion die Viskosität der äußeren Phase erhöht, die Diffusion der Öltropfen verringert und damit die Langzeitstabilität erhöht. In einer wasserfreien Formulierung ist dies nicht möglich. Des Weiteren ist das Erhöhen der Viskosität lipophiler Phasen schwieriger da hier weniger Mechanismen zur Verfügung stehen. Im Allgemeinen werden dann polymere Ölverdicker oder Feststoffe hinzu gegeben. Erfindungsgemäß bevorzugt wird zur Viskositätsverbesserung die Zugabe von Wachskomponenten, insbesondere zwei verschiedene Wachse, gewählt. Überraschenderweise führt die Kombination zweier Wachse hier zu einem deutlich höheren Effekt, als die gleiche Konzentration der Einzelwachskomponenten.

In einer weiteren bevorzugten Ausführungsform werden den erfindungsgemäßen Zubereitungen hydrogenated Castor oil in Kombination mit Magnesiumstearat hinzugefügt.

Die Kombination verbessert die Stabilität der Zubereitung abermals durch deutlich effizientere Viskositätserhöhung. Wohingegen die jeweiligen Einzelkomponenten dies nicht im gleichen Maße erzielen.

In einer Vergleichsuntersuchungen hat der Zusatz von 3 Gew.% Magnesiumstearat keinerlei Verdickungswirkungen der erfindungsgemäßen Zubereitungen bewirkt.

Der Zusatz von 3 Gew.% hydrogenated Castor oil führte zu einer Viskositätserhöhung der Zubereitung auf 3400 mPas (25°C).

Der Zusatz von 1,5 Gew.% Magnesiumstearat und 1,5 Gew.% hydrogenated Castor Oil führte überraschenderweise aber zu einer Viskosität der erfindungsgemäßen Zubereitungen von bis zu 11700 mPas (25°C).

Dies bedeutet, dass die Kombination zu einem synergistische Effekt führt, der deutlich effizienter die Viskosität erhöht und damit die Stabilität. Dies ermöglicht ein wesentlich feineres und breiteres Spektrum an maßgeschneiderter Sensorik, da Wachse generell einen starken Einfluß auf die Sensorik haben.

Es werden daher den erfindungsgemäßen Zubereitungen bevorzugt 0,2 Gew.% bis 2 Gew.% an hydrogenated Castor oil und 0,2 bis 2 Gew.% Magnesiumstearat zugesetzt, jeweils bezogen auf die Gesamtmasse der Zubereitung.

Dabei ist das Gewichtsverhältnis der beiden Substanzen hydrogenated Castor Oil zu Magnesiumstearat bevorzugt zwischen 0,75 : 1,25 bis 1,25 : 0,75 zu wählen.

f) Siloxanelastomere

In einer bevorzugten Ausführungsform umfasst die erfindungsgemäße Zubereitung ein oder mehrere Siloxanelastomere. Bevorzugt sind ein oder mehrere Elastomere zu wählen im Anteilsbereich von 0,1 Gew.% bis 12 Gew.%, bezogen auf die Gesamtmasse der Zubereitung. Erstaunlicherweise ist das Trocknungsverhalten durch die Zugabe an einem oder mehreren Siloxnelastomeren nochmals verbessert.

In einer Vergleichsuntersuchung zeigte sich, dass erfindungsgemäße Zubereitungen mit dem Zusatz an Siloxanelastomer und Dimethicon die Trocknungskinetik verbessern, obwohl keine Wasseraufnahme durch die Dimethicone erfolgt.

Zwei erfindungsgemäße Zubereitungen, die sich lediglich dadurch unterschieden, dass die eine Zubereitung ein Siloxanelastomer und die andere Zubereitung kein Elastomer umfasste, wurden durch ein zehnköpfiges Expertenpanel unter standardisierten Bedingungen verglichen.

| M 685 | N 701 | inci |
|---|---|---|
| 5,12 | 7,21 | Isoamyl Laurate |
| 5 | 5 | Carboxymethylcellulose |
| 3 | 3 | Hydrogenated Castor Oil |
| 15,74 | 22,25 | Isopropyl Palmitate |
| 1,67 | 2,37 | Octyldodecanol |
| 1,67 | 2,37 | C12-15 Alkyl Benzoate |
| 20 | 0 | Dimethicone |
| 1 | 1 | Diisostearoyl Polyglyceryl-3 Dimer Dilinoleate |
| 1 | 1 | Polyglyceryl-4 Diisostearate/Polyhydroxystearate/Sebacate |
| 5 | 5 | Calcium Starch Octenylsuccinate + Aqua + Calcium Chloride |
| 0 | 10 | Dimethicone + Dimethicone Crosspolymer |
| 0,8 | 0,8 | Parfum |
| 6 | 6 | Glycerin |

(fortgesetzt)

| M 685 | N 701 | inci |
|---|---|---|
| 4 | 4 | Magnesium Stearate |
| 30 | 30 | Alcohol Denat. + Aqua |

Ergebnis des Panel: Die Mehrheit der Personen fanden N701 deutlich trockener als M685

g) Hautbefeuchtungsmittel

Den erfindungsgemäßen Zubereitungen können ein oder mehrere Hautbefeuchtungsmittel zugesetzt werden. Es zeigte sich, dass aufgrund des Zusatzes an Hautbefeuchtungsmitteln (moisturizern), die Sensorik, Haptik und vor allem die Pflegeeigenschaften der erfindungsgemäßen Zubereitungen nochmals verbessert werden kann.

Vorteilhaft werden den Zubereitungen 1 Gew.% bis zu 30 Gew.%, insbesondere 1 Gew.% bis 20 Gew.%, bezogen auf die Gesamtmasse der Zubereitung, eines oder mehrerer Hautbefeuchtungsmittel zugesetzt. Vorteilhaft wird Glycerin als Befeuchtungsmittel gewählt.

Einen hohen Anteil an Glycerin im Bereich bis zu 30 Gew.% zu wählen hat den Vorteil, dass hohe Mengen Glycerin einen Wärmeeffekt erzeugen, der für den Anwender angenehm sein kann.

h) Weitere zusätzlich enthaltene Substanzen

Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Substanzen zum Verhindern des Schäumens, Farbstoffe und Farbpigmente, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate, Selbstbräuner, Puffer, pH Regulatoren, Pflanzliche Extrakte, Sebumabsorbierende Substanzen, UV-Filter, Wirkstoffe wie zum Beispiel Anti Age, Anti-Cellulite, Anti Akne, Anti-Rosacea, Anti-Neurodermitis, Antioxidantien, Moisturiser, Chelatbildner, Antitranspirantien, Bleich- und Färbemittel, Parfum etc. sofern der Zusatz die geforderten Eigenschaften hinsichtlich der Wasseraufnahme, Stabilität und sensorische Eigenschaften nicht behindert oder erfindungsgemäß ausgeschlossen sind.

Als mögliche zusätzliche Substanzen können den erfindungsgemäßen Zubereitungen bevorzugt hinzugefügt werden:

1. Volatile Substanzen, wie insbesondere Ethanol, Cyclomethicone, kurzkettige Dimethicone, kurzkettige Parafine, Isodecyl Neopentanoate, Dibutyl Adipate und andere Substanzen mit vergleichbarem Voltitlitätsprofil

Vorteil: der Zusatz führt zu einem leichteren Hautgefühl, der Möglichkeit der Steuerung der Sensorik und einer gefühlt besseren Trockenheit der Haut des Konsumenten

1. Sensorikadditive, insbesondere Kohlehydrate, zum Beispiel Stärke oder Cellulose Derivate, Silikonelastomere und pudrige Rohstoffe, Füllstoffe (polymere und nicht polymere) welche nicht unter die Gruppe der Superabsorber fallen.

Vorteile: der Zusatz führt zu einer angenehmeren Sensorik und der Möglichkeit der Steuerung der Sensorik.

2. Ölverdicker

Vorteile: der Zusatz führt zur Erhöhung der Viskosität, so dass beispielsweise die Zubereitung dem Anwender nicht durch die Finger rinnt oder eine erhöhte Langzeitstabilität aufweist.

3. Filmbildner

Vorteile: der Zusatz führt zur Verbesserung der Wasserfestigkeit. Insbesondere bei Lichtschutzzubereitungen ist dies ein für den Anwender wesentliche Eigenschaft um nach dem baden sich nicht ständig neu eincremen zu müssen. Es ist jedoch auch erfindungsgemäß in einer anderen Ausführungsform gänzlich auf Filmbildner zu verzichten.

Anwendungsformen

Als mögliche Anwendungsformen sind für die erfindungsgemäßen Zubereitungen zunächst alle gängigen Formen geeignet, wodurch die erfindungsgemäßen Zubereitungen einen weiteren Formulierungsvorteil bei der Herstellung bieten.
Bevorzugt sind flüssige, gelförmige und/oder semisolide Formen.

Verwendung

Die Erfindung umfasst die Verwendung einer topisch applizierbaren Zubereitung umfassend

a) ein oder mehrere Lipide,
b) mindestens einen W/O Emulgator und
c) mindestens 0,05 Gew.% eines oder mehrerer Superabsorber, bezogen auf die Gesamtmasse der Zubereitung,

zur Trocknung und zeitgleichen Pflege der Haut.
Die erfindungsgemäße Zubereitung lässt sich auf die noch nasse oder feuchte Haut auftragen, verteilen und führt so zu einer Trocknung und zeitgleichen Pflege der Haut. Bei der Trocknung ist nicht eine Hautaustrocknung sondern lediglich die Reduzierung der Feuchte, Nässe zu verstehen, die zuvor auf der Haut vorhanden war.
Weitere erfindungsgemäße Verwendungen der erfindungsgemäßen Zubereitungen ergeben sich aus den optional zugesetzten Stoffen.
Die nachfolgenden Beispiele erläutern erfindungsgemäße Zubereitungen. Die Zahlen sind, sofern nichts anderes angegeben, Gewichtsanteile bezogen auf die Gesamtmasse der Zubereitung.

Beispielrezepturen:

| INCI | 1 | 2 | 3 | 4 | 5 | 6 | 7 | 8 |
|---|---|---|---|---|---|---|---|---|
| Silica Silylate | 0 | 1 | 0 | 2 | 1,5 | 0 | 1,5 | 1,5 |
| Sodium starch glycolate | 5 | 7 | 5 | 3 | 7 | 5 | 7 | 7 |
| Isoamyl Laurate | 7 | 7 | | 10 | | 6 | 7 | 7 |
| Hydrogenated Castor Oil | 3 | 3 | 2 | 3 | 4 | 3 | 3 | 3 |
| Magnesium Stearate | 4 | 4 | 3 | 4 | 5 | 4 | 4 | 4 |
| Octyldodecanol | | 2 | 5 | 1 | 2,5 | | 2 | 2 |
| C12-15 Alkyl Benzoate | 2,5 | 2 | 2,5 | 2 | 3 | 2,5 | 4 | 4 |
| Isopropyl Palmitate | 25 | | 20 | | 25 | 20 | 22 | |
| Cyclomethicone | 5 | | 10 | | | | | |
| Isodecyl Neopentanoate | | | | 20 | | | | |
| C13-16 Isoparaffin | | | | | 10 | | | |
| Simmondsia Chinensis Seed Oil | 10 | | | | | 10 | | |
| PPG-15 Stearyl Ether | | | | | | | 10 | |
| Butylene Glycol Dicaprylate/ Dicaprate | | | | | | | | 22 |
| Paraffinum Liquidum | | 20 | | | | | | |
| Diisostearoyl Polyglyceryl-3 Dimer Dilinoleate | 3 | 1 | 2 | 1 | 1 | 2 | 1 | 1 |
| Polyglyceryl-4 Diisostearate/ Polyhydroxystearate/Sebaca te | 3 | 1 | 2 | 1 | 1 | 2 | 1 | 1 |

(fortgesetzt)

| INCI | 1 | 2 | 3 | 4 | 5 | 6 | 7 | 8 |
|---|---|---|---|---|---|---|---|---|
| Calcium Starch Octenylsuccinate + Aqua + Calcium Chloride | 6 | 2 | 5 | 4 | 5 | 7 | 2 | 2 |
| Dimethicone + Dimethicone Crosspolymer | | 10 | | | | 10 | 10 | 10 |
| Parfum | 0,8 | 0,2 5 | 0,7 | 0,3 | 0,8 | 0,5 | 1 | 1 |
| Glycerin | 5 | 5 | 0 | 10 | 8 | 6 | 3 | 3 |
| Alcohol Denat. + Aqua | ad 100 | ad 100 | ad 100 | ad 100 | ad 100 | ad 100 | ad 100 | ad 100 |

Beispielrezepturen mit UV Filter

| | 1 | 2 | 3 | 4 | 5 | 6 | 7 | 8 | 9 |
|---|---|---|---|---|---|---|---|---|---|
| Silica Silylate | 2 | 1 | 2 | 2 | 3 | 0 | 2 | 0,5 | 1 |
| Sodium starch glycolate | 5 | 4 | 0 | 4 | 8 | 6 | 2 | 5 | 3 |
| Isoamyl Laurate | 0 | 0 | 0 | 0 | 0 | 0 | 0 | 0 | 0 |
| Hydrogenated Castor Oil | 3 | 3 | 3 | 3 | 3 | 3 | 3 | 3 | 3 |
| Magnesium Stearate | 0 | 0 | 0 | 0 | 0 | 0 | 0 | 0 | 0 |
| Octyldodecanol | 1 | 0 | 2 | 0 | 0 | 0 | 4 | 0 | 0 |
| Isopropyl Palmitate | 9,5 | 0 | 0 | 0 | 0 | 0 | 0 | 0 | 0 |
| Dibutyl Adipate | 3 | 3 | 3 | 3 | 3 | 3 | 10 | 3 | 3 |
| C12-15 Alkyl Benzoate | 0 | 9,5 | 3 | 0 | 0 | 0 | 0 | 0 | 0 |
| Cyclomethicone | 0 | 0 | 0 | 0 | 0 | 9,5 | 0 | 0 | 0 |
| Isodecyl Neopentanoate | 0 | 0 | 0 | 0 | 0 | 0 | 0 | 12 | 0 |
| C13-16 Isoparaffin | 0 | 0 | 0 | 0 | 0 | 0 | 0 | 0 | 10 |
| Simmondsia Chinensis Seed Oil | 0 | 0 | 0 | 0 | 7 | 0 | 0 | 0 | 0 |
| PPG-15 Stearyl Ether | 0 | 0 | 0 | 9 | 0 | 0 | 0 | 0 | 0 |
| Butylene Glycol Dicaprylate/ Dicaprate | 3 | 3 | 10 | 3 | 3 | 3 | 3 | 3 | 3 |
| Cera Alba | 3 | 3 | 3 | 3 | 3 | 3 | 3 | 3 | 3 |
| Diisostearoyl Polyglyceryl-3 Dimer Dilinoleate | 0,5 | 0,5 | 0,5 | 0,5 | 0,5 | 0,5 | 0,5 | 0,5 | 0,5 |
| Polyglyceryl-4 Diisostearate/ Polyhydroxystea rate/ Sebacate | 0,5 | 0,5 | 0,5 | 0,5 | 0,5 | 0,5 | 0,5 | 0,5 | 0,5 |
| Calcium Starch Octenylsuccinate + Aqua + Calcium Chloride | 4 | 5 | 5 | 5 | 3 | 7 | 5 | 5 | 5 |
| Dimethicone + Dimethicone Crosspolymer | | 5 | | 5 | | 5 | 5 | | |
| Parfum | 0,3 | 0,7 | 1 | 0,5 | 0,7 | 0,6 | 9 | 0,7 | 0,3 |

(fortgesetzt)

| | 1 | 2 | 3 | 4 | 5 | 6 | 7 | 8 | 9 |
|---|---|---|---|---|---|---|---|---|---|
| Glycerin | 2 | 2,5 | 3 | 2,5 | 7 | 2,5 | 6 | 2,5 | 4 |
| Alcohol Denat. + Aqua | ad 100 | ad 100 | ad 100 | ad 100 | ad 100 | ad 100 | ad 100 | ad 100 | ad 100 |
| Homosalate | 0 | 0 | 0 | 0 | 9 | 9 | 0 | 9 | 0 |
| Octocrylene | 0 | 0 | 8 | 0 | 9 | 9 | 0 | 9 | 0 |
| Ethylhexyl Salicylate | 2 | 4,5 | | 5 | 4,5 | 4,5 | 4,5 | 4,5 | 4,5 |
| Bis-Ethylhexyloxyphenol Methoxyphenyl Triazine | 3,5 | 2 | 3,5 | 2,5 | 2 | 2 | 2 | 2 | 2 |
| Butyl Methoxydibenzoylmethane | 4,8 | 4 | 4,5 | 3 | 4,5 | 4,5 | 4 | 4,5 | 4 |
| Polysilicone-15 | 0 | 0 | 0 | 0 | 1 | 0 | 0 | 1 | 0 |
| Diethylamino Hydroxybenzoyl Hexyl Benzoate | 1 | 0 | 0 | 0 | 0 | 0 | 0 | 0 | 0 |
| Ethylhexyl Triazone | 0,75 | 1,5 | 0 | 2 | 0 | 0 | 1,5 | 0 | 1,5 |
| Titanium Dioxide (nano) | 2 | 0,5 | 6 | 1 | 0 | 0 | 3 | 0 | 3 |
| Phenylbenzimidazole Sulfonic Acid | 0 | 1 | 0 | 0 | 0 | 0 | 1 | 5 | 1 |
| Zinc Oxide | 0 | 0 | 0 | 0 | 0 | 0 | 3 | 0 | 3 |

| INCI | 10 |
|---|---|
| Silica Silylate | 2 |
| Carboxymethylstärke (Sodium starch glycolate) | 5 |
| Dibutyl Adipate | 3 |
| C12-15 Alkyl Benzoate | 9,5 |
| Hydrogenated Castor Oil | 3 |
| Butylene Glycol Dicaprylate/Dicaprate | 3 |
| Cera Alba | 3 |
| Diisostearoyl Polyglyceryl-3 Dimer Dilinoleate | 0,5 |
| Polyglyceryl-4 Diisostearate/Polyhydroxystearate/Sebacate | 0,5 |
| Calcium Starch Octenylsuccinate + Aqua + Calcium Chloride | 5 |
| Dimethicone + Dimethicone Crosspolymer | 5 |
| Parfum | 0,7 |
| Glycerin + Aqua | 2,5 |
| Alcohol Denat. + Aqua | 27,3 |
| Homosalate | 9 |
| Octocrylene | 9 |
| Ethylhexyl Salicylate | 4,5 |
| Bis-Ethylhexyloxyphenol Methoxyphenyl Triazine | 2 |

(fortgesetzt)

| Butyl Methoxydibenzoylmethane | 4,5 |
|---|---|
| Polysilicone-15 | 1 |

**Patentansprüche**

1. Kosmetische oder dermatologische Zubereitung umfassend

   a) ein oder mehrere Lipide,
   b) mindestens einen W/O Emulgator
   c) mindestens 0,05 Gew.% eines oder mehrerer Superabsorber, bezogen auf die Gesamtmasse der Zubereitung,

   **dadurch gekennzeichnet, dass**
   die Zubereitung maximal 1 Gew.% an Wasser, vorteilhaft weniger als 0,3 Gew.% Wasser umfasst, bezogen auf die Gesamtmasse der Zubereitung.

2. Zubereitung nach Anspruch 1 **dadurch gekennzeichnet, dass** die Zubereitung nur

   a) ein oder mehrere Lipide,
   b) mindestens einen W/O Emulgator und
   c) mindestens 0,05 Gew.% eines oder mehrerer Superabsorber, bezogen auf die Gesamtmasse der Zubereitung, umfasst.

3. Zubereitung nach einem der vorstehenden Ansprüche **dadurch gekennzeichnet, dass** das oder die Lipide einen nach der in der Beschreibung angegebenen Methode gemessenen Spreitwert über 400 aufweisen.

4. Zubereitung nach einem der vorstehenden Ansprüche **dadurch gekennzeichnet, dass** das oder die Lipide gewählt werden aus der Gruppe Dibutyl Adipate, Isopropyl Myristate Isodecyl Neopentanoate, Octyldodecanol, Caprylic/Capric Triglyceride, Propylene Glycol Dicaprylate/Dicaprate, Dicaprylyl Ether, Paraffinum Liquidum, Isopropyl Palmitate, C13-16 Isoparaffin, Isohexadecane, Cyclomethicone, Hydrogenated Polydecene, Octyldodecyl Myristate, Decyl Oleate, Butylene Glycol Dicaprylate/Dicaprate, Dicaprylyl Carbonate, Hydrogenated Polyisobutene, Isopropyl Isostearate, Cetearyl Isononanoate, Isopropyl Stearate, Phenyl Trimethicone, C12-15 Alkyl Benzoate, Ethylhexyl Cocoate, Squalane, Ethylhexyl Stearate, Cetearyl Ethylhexanoate+Isopropyl Myristate, C12-13 Alkyl Lactate, Coco-Caprylate/Caprate, Persea Gratissima Oil, Simmondsia Chinensis Seed Oil.

5. Zubereitung nach einem der vorstehenden Ansprüche **dadurch gekennzeichnet, dass** als Lipide ein oder mehrere Wachse gewählt werden mit einem Schmelzpunkt im Bereich zwischen 30 bis 120 °C, bevorzugt mit einem Schmelzpunkt im Bereich 40°C bis 100°C, besonders bevorzugt im Bereich 40°C bis 90°C.

6. Zubereitung nach Anspruch 5 **dadurch gekennzeichnet, dass** der Anteil an Wachsen im Bereich von 1 Gew.% bis 14 Gew.%, bevorzugt 2 Gew.% bis 12 Gew.%, besonders bevorzugt 4 Gew.% bis 10 Gew.%, gewählt wird, bezogen auf die Gesamtmasse der Zubereitung.

7. Zubereitung nach einem der vorstehenden Ansprüche **dadurch gekennzeichnet, dass** der Anteil an einem oder mehreren Lipiden im Bereich von 20 Gew.% bis 75 Gew.%, bevorzugt im Bereich 35 Gew.% bis 65 Gew.%, besonders bevorzugt im Bereich von 40 Gew.% bis 60 Gew.%, gewählt wird, bezogen auf die Gesamtmasse der Zubereitung.

8. Zubereitung nach einem der vorstehenden Ansprüche **dadurch gekennzeichnet, dass** ein oder mehrere W/O-Emulgatoren aus der Gruppe Polyglyceryl-n Diisostearate, Polyglyceryl-n-dipolyhydroxystearate, insbesondere Polyglyceryl-n Dipolyhydroxystearate, Diisostearoyl Polyglyceryl-n Dimer Dilinoleate und/oder Polyglyceryl-n Diisostearate/Polyhydroxystearate/ Sebacate gewält werden.

9. Zubereitung nach einem der vorstehenden Ansprüche **dadurch gekennzeichnet, dass** der Anteil an W/O-Emulgatoren 0,25 bis 10 Gew.%, insbesondere 0,75 bis 7 Gew.%, beträgt, bezogen auf die Gesamtmasse der Zubereitung.

**10.** Zubereitung nach einem der vorstehenden Ansprüche **dadurch gekennzeichnet, dass** der oder die Superabsorber eine Wasseraufnahmekapazität des bis zu 1000-fachen ihres Gewichts, bevorzugt bis zu 200-fachen ihres Gewichts, insbesondere im Bereich vom 50-fachen ihres Gewichtes bis zu dem 5-fachen ihres Gewichtes, aufweist.

**11.** Zubereitung nach einem der vorstehenden Ansprüche **dadurch gekennzeichnet, dass** der oder die Superabsorber gewählt werden aus der Gruppe der Carboxymethylcellulosen und Carboxymethylstärken, insbesondere Natriumstärkeglycolat und/oder Natriumcelluloseglycolat.

**12.** Zubereitung nach einem der vorstehenden Ansprüche **dadurch gekennzeichnet, dass** der Anteil an einem oder mehreren Superabsorbern im Bereich von 0,05 bis 20 Gew.%, bevorzugt 0,1 bis 10 Gew.%, beträgt, bezogen auf die Gesamtmasse der Zubereitung.

**13.** Zubereitung nach einem der vorstehenden Ansprüche 1 oder 3 bis 12 umfassend zusätzlich ein oder mehrere UV-Filter.

**14.** Zubereitung nach einem der vorstehenden Ansprüche 1 oder 3 bis 13 umfassend hydrogenated Castor oil und Magnesiumstearat.

**15.** Zubereitung nach einem der vorstehenden Ansprüche 1 oder 3 bis 14 umfassend ein oder mehrere Siloxanelastomere.

**16.** Zubereitung nach einem der vorstehenden Ansprüche vorstehenden Ansprüche 1 oder 3 bis 15 umfassend ein oder mehrere Stoffe gewählt aus der Gruppe Ethanol, Cyclomethicone, kurzkettige Dimethicone, kurzkettige Parafine, Isodecyl Neopentanoate und/oder Dibutyl Adipate.

**17.** Nichttherapeutische Verwendung der Zubereitung nach einem der vorstehenden Ansprüche zur Trocknung und zeitgleichen Pflege der Haut.

**18.** Zubereitung nach einem der Ansprüche 13 bis 16 zur Anwendung in einem Verfahren zum Schutz der Haut gegen UV Strahlung bei zeitgleicher Trocknung der Haut, wobei die Zubereitung auf nasser Haut appliziert wird.

**Claims**

**1.** Cosmetic or dermatological preparation comprising

a) one or more lipids,
b) at least one W/O emulsifier
c) at least 0.05% by weight of one or more superabsorbents, based on the total mass of the preparation,

**characterized in that**
the preparation comprises a maximum of 1% by weight of water, advantageously less than 0.3% by weight water, based on the total mass of the preparation.

**2.** Preparation according to Claim 1, **characterized in that** the preparation comprises only

a) one or more lipids,
b) at least one W/O emulsifier and
c) at least 0.05% by weight of one or more superabsorbents, based on the total mass of the preparation.

**3.** Preparation according to either of the preceding claims, **characterized in that** the lipid or lipids have a spreading value, measured by the method stated in the description, above 400.

**4.** Preparation according to any of the preceding claims, **characterized in that** the lipid or lipids are selected from the group comprising dibutyl adipate, isopropyl myristate, isodecyl neopentanoate, octyldodecanol, caprylic/capric triglycerides, propylene glycol dicaprylate/dicaprate, dicaprylyl ether, paraffinum liquidum, isopropyl palmitate, C13-16 isoparaffin, isohexadecane, cyclomethicone, hydrogenated polydecene, octyldodecyl myristate, decyl oleate, butylene glycol dicaprylate/dicaprate, dicaprylyl carbonate, hydrogenated polyisobutene, isopropyl isostearate, cetearyl isononanate, isopropyl stearate, phenyl trimethicone, C12-15 alkyl benzoate, ethylhexyl cocoate, squalane, ethylhexyl stearate, cetearyl ethylhexanoate + isopropyl myristate, C12-13 alkyl lactate, coco-caprylate/caprate, Persea gratissima oil, Simmondsia chinensis seed oil.

5. Preparation according to any of the preceding claims, **characterized in that** one or more waxes are selected as lipids having a melting point in the range between 30 to 120°C, preferably having a melting point in the range 40°C to 100°C, particularly preferably in the range 40°C to 90°C.

6. Preparation according to Claim 5, **characterized in that** the proportion of waxes is selected in the range of 1% by weight to 14% by weight, preferably 2% by weight to 12% by weight, particularly preferably 4% by weight to 10% by weight, based on the total mass of the preparation.

7. Preparation according to any of the preceding claims, **characterized in that** the proportion of one or more lipids is selected in the range of 20% by weight to 75% by weight, preferably in the range of 35% by weight to 65% by weight, particularly preferably in the range of 40% by weight to 60% by weight, based on the total mass of the preparation.

8. Preparation according to any of the preceding claims, **characterized in that** one or more W/O emulsifiers are selected from the group comprising polyglyceryl-n diisostearates, polyglyceryl-n dipolyhydroxystearates, particularly polyglyceryl-n dipolyhydroxystearates, diisostearoyl polyglyceryl-n dimer dilinoleates and/or polyglyceryl-n diisostearates/polyhydroxystearates/sebacates.

9. Preparation according to any of the preceding claims, **characterized in that** the proportion of W/O emulsifiers is 0.25 to 10% by weight, in particular 0.75 to 7% by weight, based on the total mass of the preparation.

10. Preparation according to any of the preceding claims, **characterized in that** the superabsorbent(s) have a water absorption capacity of up to 1000 times its weight, preferably up to 200 times its weight, in particular in the range from 50 times its weight up to 5 times its weight.

11. Preparation according to any of the preceding claims, **characterized in that** the superabsorbent(s) are selected from the group of carboxymethyl celluloses and carboxymethyl starches, in particular sodium starch glycolate and/or sodium cellulose glycolate.

12. Preparation according to any of the preceding claims, **characterized in that** the proportion of one or more superabsorbents is in the range from 0.05 to 20% by weight, preferably 0.1 to 10% by weight, based on the total mass of the preparation.

13. Preparation according to any of the preceding Claims 1 or 3 to 12 additionally comprising one or more UV filters.

14. Preparation according to any of the preceding Claims 1 or 3 to 13 comprising hydrogenated castor oil and magnesium stearate.

15. Preparation according to any of the preceding Claims 1 or 3 to 14 comprising one or more siloxane elastomers.

16. Preparation according to any of the preceding Claims 1 or 3 to 15 comprising one or more substances selected from the group comprising ethanol, cyclomethicones, short-chain dimethicones, short-chain parafins, isodecyl neopentanoate and/or dibutyl adipate.

17. Non-therapeutic use of the preparation according to any of the preceding claims for drying and simultaneous care of the skin.

18. Preparation according to any of Claims 13 to 16 for use in a method for protecting the skin from UV radiation and at the same time drying the skin, wherein the preparation is applied to wet skin.

**Revendications**

1. Préparation cosmétique ou dermatologique comprenant

   a) un ou plusieurs lipides,
   b) au moins un émulsifiant E/H
   c) au moins 0,05 % en poids d'un ou plusieurs superabsorbants, par rapport à la masse totale de la préparation,
   **caractérisée en ce que** la préparation comprend au maximum 1 % en poids d'eau, préférablement moins de 0,3 % en poids d'eau, par rapport à la masse totale de la préparation.

2. Préparation selon la revendication 1, **caractérisée en ce que** la préparation comprend uniquement

   a) un ou plusieurs lipides,
   b) au moins un émulsifiant E/H et
   c) au moins 0,05 % en poids d'un ou plusieurs superabsorbants, par rapport à la masse totale de la préparation.

3. Préparation selon l'une des revendications précédentes, **caractérisée en ce que** le ou les lipides présentent un indice d'étalement supérieur à 400, mesuré selon la méthode indiquée dans la description.

4. Préparation selon l'une des revendications précédentes, **caractérisée en ce que** le ou les lipides sont choisis dans le groupe comprenant l'adipate de dibutyle, le myristate d'isopropyle, le néopentanoate d'isodécyle, l'octyldodécanol, les triglycérides capryliques/capriques, le dicaprylate/dicaprate de propylène glycol, l'éther dicaprylylique, l'huile de paraffine, le palmitate d'isopropyle, l'isoparaffine en $C_{13}$-$C_{16}$, l' isohexadécane, le cyclométhicone, le polydécène hydrogéné, le myristate d'octyldocéyle, l'oléate de décyle, le dicaprylate/dicaprate de butylène glycol, le carbonate de dicaprylyle, le polyisobutène hydrogéné, l'isostéarate d'isopropyle, l'isononanoate de cétéaryle, le stéarate d'isopropyle, le phényl triméthicone, un benzoate d'alkyle en $C_{12}$-$C_{15}$, le cocoate d'éthylhexyle, le squalane, le stéarate d'éthylhexyle, l'éthylhexanoate de cétéaryle + le myristate d'isopropyle, le lactate d'alkyle en $C_{12}$-$C_{13}$, le coco-caprylate/caprate, l'huile de Persea Gratissima, l'huile de graines de Simmondsia Chinensis.

5. Préparation selon l'une des revendications précédentes, **caractérisée en ce que** l'on choisit comme lipides une ou plusieurs cires ayant un point de fusion dans la plage de 30, à 120 °C, préférablement ayant un point de fusion dans la plage de 40 °C à 100 °C, particulièrement préférablement dans la plage de 40 °C à 90 °C.

6. Préparation selon la revendication 5, **caractérisée en ce que** la proportion de cires est choisie dans la plage de 1 % en poids à 14 % en poids, préférablement de 2 % en poids à 12 % en poids, particulièrement préférablement de 4 % en poids à 10 % en poids, par rapport à la masse totale de la préparation.

7. Préparation selon l'une des revendications précédentes, **caractérisée en ce que** la proportion d'un ou plusieurs lipides se situe dans la plage de 20 % en poids à 75 % en poids, préférablement de 35 % en poids à 65 % en poids, particulièrement préférablement dans la plage de 40 % en poids à 60 % en poids, par rapport à la masse totale de la préparation.

8. Préparation selon l'une des revendications précédentes, **caractérisée en ce qu'**un ou plusieurs émulsifiants E/H du groupe comprenant le diisostéarate de polyglycéryl-n, le dipolyhydroxystéarate de polyglycéryl-n, en particulier le dipolyhydroxystéarate de polyglycéryl-n, le dimère de dilinoléate de diisostéaroyl polyglycéryl-n et/ou le diisostéarate/polyhydroxy-stéarate/sébaçate de polyglycéryl-n.

9. Préparation selon l'une des revendications précédentes, **caractérisée en ce que** la proportion d'émulsifiants E/H est de 0,25 à 10 % en poids, en particulier de 0,75 à 7 % en poids, par rapport à la masse totale de la préparation.

10. Préparation selon l'une des revendications précédentes, **caractérisée en ce que** le ou les superabsorbants présentent une capacité d'absorption de l'eau allant jusqu'à 1 000 fois leur poids, préférablement jusqu'à 200 fois leur poids, en particulier dans la plage de 50 fois leur poids à 5 fois leur poids.

11. Préparation selon l'une des revendications précédentes, **caractérisée en ce que** le ou les superabsorbants sont choisis dans le groupe comprenant les carboxyméthylcelluloses et les carboxyméthylamidons, en particulier le glycolate d'amidon sodique et/ou le glycolate de cellulose sodique.

**12.** Préparation selon l'une des revendications précédentes, **caractérisée en ce que** la proportion d'un ou plusieurs superabsorbants se situe dans la plage de 0,05 à 20 % en poids, préférablement de 0,1 à 10 % en poids, par rapport à la masse totale de la préparation.

**13.** Préparation selon l'une des revendications précédentes 1 ou 3 à 12 comprenant en outre un ou plusieurs filtres UV.

**14.** Préparation selon l'une des revendications précédentes 1 ou 3 à 13 comprenant en outre de l'huile de ricin hydrogénée ou du stéarate de magnésium.

**15.** Préparation selon l'une des revendications précédentes 1 ou 3 à 14 comprenant en outre un ou plusieurs élastomères siloxanes.

**16.** Préparation selon l'une des revendications précédentes 1 ou 3 à 15 comprenant en outre une ou plusieurs substances choisies dans le groupe comprenant l'éthanol, le cyclométhicone, le diméthicone à chaîne courte, des paraffines à chaîne courte, le néopentanoate d'isodécyle et/ou l'adipate de dibutyle.

**17.** Utilisation non thérapeutique de la préparation selon l'une des revendications précédentes pour le séchage et le soin simultané de la peau.

**18.** Préparation selon l'une des revendications 13 à 16 pour une utilisation dans un procédé pour protéger la peau contre le rayonnement UV et sécher simultanément la peau, la préparation étant appliquée sur peau humide.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2023888 A2
- FR 2999919

- US 2013338052 A
- EP 2819639 A2

**In der Beschreibung aufgeführte Nicht-Patentliteratur**

- *CHEMICAL ABSTRACTS,* 288254-16-0